# EUROPEAN PATENT APPLICATION

(11) **EP 3 446 662 A1**
(43) Date of publication of application: **27.02.2019**
(21) Application number: 17785970.9
(22) Date of filing: 18.04.2017
(51) Int. Cl.: A61F 5/045, A61F 5/01

(54) **MEDICAL DEVICE**

(30) Priority: 19.04.2016 JP 2016083917
(71) Applicant: MIZUHO Corporation, Tokyo 113-0033 (JP)
(72) Inventor: EBARA Sohei, Chigasaki-shi Kanagawa 253-0013 (JP)
(74) Representative: SSM Sandmair
(86) International application number: PCT/JP2017/015565
(87) International publication number: WO 2017/183632

(57) **Abstract**

Provided is a medical device whereby a burden placed on a patient in spinal deformity surgery is alleviated while facilitating the surgery. A medical device in which a chest unit 5A for supporting the chest of a patient 2 and a pelvic unit 5B for supporting the pelvis of the patient 2 are disposed side by side on a base 10, the medical device being provided with a first shifting device 15, 16 for shifting the chest unit 5A in a first direction closer to or away from the pelvic unit 5B, and a second shifting device 25, 46 for shifting either the chest unit 5A or the pelvic unit 5B in a horizontal direction orthogonal to the first direction with respect to the other of the chest unit 5A or the pelvic unit 5B.

## Description

### TECHNICAL FIELD

The present invention relates to a medical device for treating spinal deformity, and it particularly relates to a spinal correction device for correcting a spine that has been deformed.

### BACKGROUND ART

Surgery for spinal deformity is to be carried out, in order to correct spinal displacement, for example, by incising the anterior portion or the posterior portion of a spine, by installing a plurality of screws into the spine and connecting rods thereto, and then by turning back, compressing or pulling the spine.

It has been conventionally known that there is a spinal correction device for correcting spinal deformity, not involving any surgery (see Patent Document 1).

A spinal correction device disclosed in Patent Document 1 includes the following mechanisms: 1) affixing a headgear to a patient, hooking the headgear, and winding rope attached to the hook with a motor so as to pull the patient's spine; and 2) providing two boards each of which supports the upper and lower body of a patient, the boards being able to rotate about three axes.

### PRIOR ART

### PATENT DOCUMENT

Patent Document 1: Japanese Patent No. 3459999

### DISCLOSURE OF INVENTION

### PROBLEMS TO BE SOLVED BY THE INVENTION

In general, as described hereinabove, surgery for spinal deformity will be carried out by incising the anterior portion or the posterior portion of a spine and by making full use of various operational techniques to correct the spinal deformity. However, the surgery not only involves a high degree of difficulty but places an excessive burden on patients. Moreover, the number of doctors available for such a surgery may be limited.

Now, in the spine correction device disclosed in Patent Document 1, a pulling operation would be carried out by pulling the headgear attached to the patient's head, so that load may also be applied to body portions other than a spine, giving the patient an unpleasant feeling in some cases.

Therefore, in order to solve such a problem, the present application aims to provide a medical device being able to lighten the burden on a patient in surgery for spinal deformity while facilitating an easier surgery.

### MEANS TO SOLVE THE PROBLEMS

In order to solve the above problem, a medical device (1) recited in a first aspect is configured that a chest unit (5A) supporting the chest of a patient (2) and a pelvic unit (5B) supporting the pelvis of the patient are arranged side by side on a base (10). Further, the medical device comprises: a first shifting device enabling the chest unit to shift closer to or away from the pelvic unit, the shift being defined as a first direction; and a second shifting device enabling either the chest unit or the pelvic unit to shift to the other in a horizontal direction orthogonal to the first direction.

In a medical device of a second aspect, according to the medical device of the first aspect, it further comprises a first holding device (18) that holds the shifted state made by the first shifting device. Further, the second shifting device shifts the pelvic unit or the chest unit in a condition that the pelvic unit or the chest unit has shifted and been held by the first shifting device.

In a medical device of a third aspect, according to the medical device of the second aspect, it further comprises a second holding device (18) that holds the shifted state made by the second shifting device.

In a medical device of a fourth aspect according to any one of the aspects 1 to 3, the chest unit has a width of a rear portion that is narrower than a width of a front portion thereof in a longitudinal direction, and the pelvic unit has a width of a front portion that is narrower than a width of a rear portion thereof in a longitudinal direction.

### EFFECTS OF THE INVENTION

The medical device of the present invention can be configured with a simple mechanism and can reduce a manufacturing cost without increasing the size thereof. Further, the medical device of the present invention has good operability in correcting spinal deformity.

### BRIEF EXPLANATION OF DRAWINGS

FIG. 1 is a perspective diagram exemplifying the outer appearance of the medical device of the present invention;
FIG. 2 is an installation example showing that a patient is mounted on the medical device, in which FIG. 2 (a) is a perspective view, FIG. 2 (b) is a side view, and FIG. 2 (c) is a plan view;
FIG. 3 is a schematic diagram of a chest unit viewed from the right side thereof;
FIG. 4 shows one example of fixation-release mechanisms provided in the chest unit, in which FIG. 4 (a) is a schematic diagram showing a fixed state, and FIG. 4 (b) is a schematic diagram showing a released state;
FIG. 5 is a schematic diagram exemplifying how the chest unit works;
FIG. 6 is a schematic diagram showing the configuration of a pelvic unit and how the pelvic unit works;
FIG. 7 is a schematic diagram exemplifying the configuration of a leg unit viewed from the left side thereof;
FIG. 8 is a schematic diagram exemplifying how the leg unit works;
FIG. 9 is a schematic diagram showing another example of the fixation-release mechanism;
FIG. 10 is a perspective diagram exemplifying the outer appearance of the medical device of a fifth embodiment;
FIG. 11 is a plan diagram of the medical device of the fifth embodiment; and
FIG. 12 is a plan diagram exemplifying how the medical device of the fifth embodiment is used.

### EMBODIMENTS FOR CARRYING OUT THE INVENTION

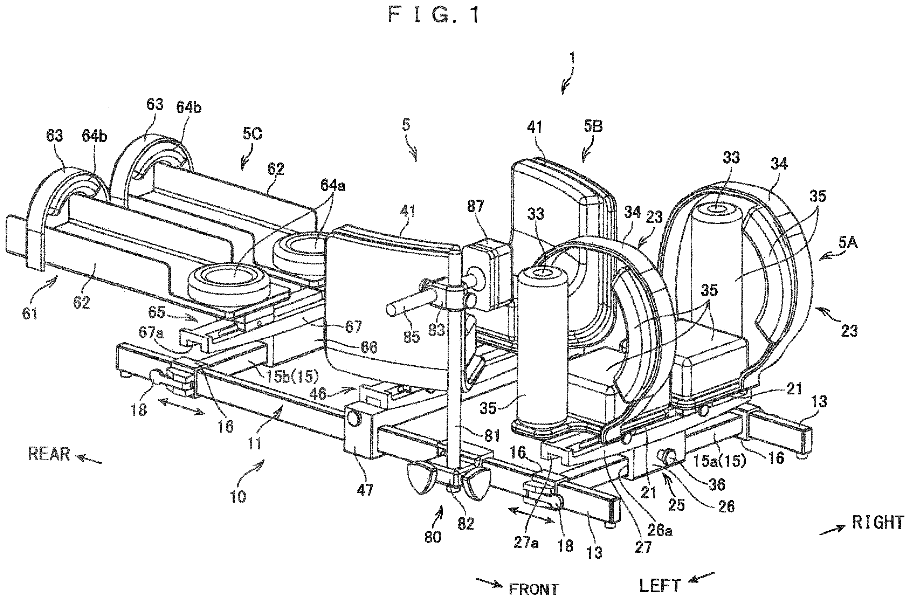

Hereinafter, the embodiments of the present invention will be explained based on some examples shown in the accompanying drawings. Although a medical device 1 shown below may be independently used as it is, it is generally placed on or fixed to an operating table or a treatment table. Further, in the medical device 1 below, the right-left direction on the sheet in FIG. 1 will be treated as the front-rear direction of the medical device for convenience.

Moreover, in the embodiments below, it is assumed that the medical device 1 is used for corrective treatment of a spine before conducting surgery for spinal deformity, but this does not prohibit it from being used during the surgery.

In general, surgery for spinal deformity needs to be performed by incision into the anterior portion or the posterior portion of a spine and by making full use of various operational techniques. Accordingly, the surgery is accompanied by a high degree of difficulty and places an excessive burden on patients. Further, the number of doctors available for the surgery may be limited.

Based on experiences in conventional spinal surgery, the present invention has revealed that it would be remarkably effective if a pulling operation is performed first to correct spinal deformity. With the medical device 1 below, treatment correcting spine displacement is first performed as much as possible. Then, surgery for the spinal deformity will be performed by incising a part of a patient's body. This can maximally simplify the surgery and greatly shorten the length of the surgery.
These steps can reduce difficulty in surgery and lessen physical burdens on the patient. Note that, in this treatment spine displacement is corrected while the patient is placed in a prone position.

As an example, this treatment will be performed before conducting surgery for spinal deformity. Specifically, a spine is first pulled in the axial direction of a patient's body, and then the chest and the pelvis are shifted in a lateral (right-left) direction, according to the deformed state of the spine, to correct the displacement of the spine. In treatment in the first embodiment described below, the pelvis is firstly positioned (fixed), and then the chest is shifted in a right-left direction according to the deformed state of the spine. Note that it would however be possible to relatively shift the chest and the pelvis in a right-left direction or to shift the pelvis while the chest is fixed. Here, according to the deformed state of the spine, the patient's legs may also shift in a right-left direction.

In the present embodiment, treatment will be conducted in advance of surgery by using the medical device 1 as described below, and surgery for spinal deformity will then be conducted.

### (First Embodiment)

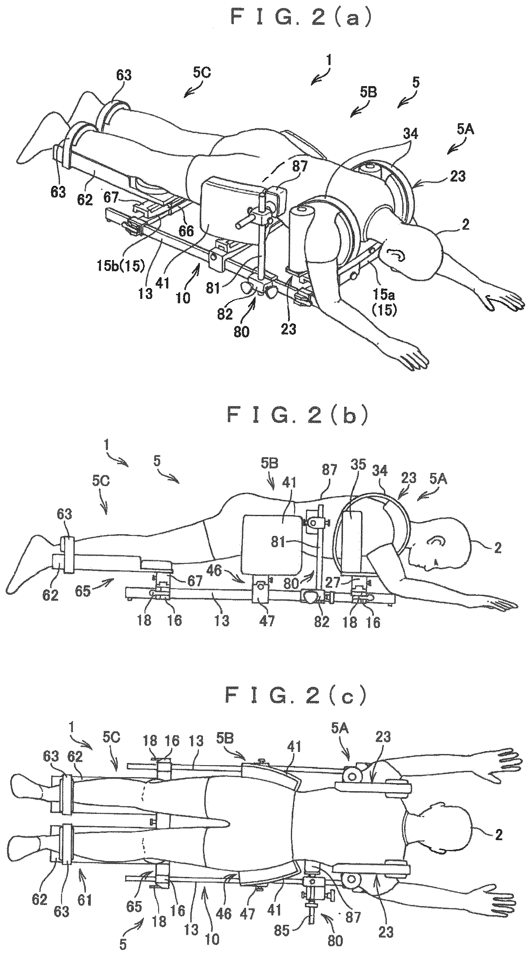

The medical device 1 shown in FIGS. 1 and 2 is used when correcting spine displacement as described above. Especially, for patients suffering from thoracic spine-deformity such as scoliosis, kyphosis and lordotic abnormality, lumbar spine-deformity or lumbosacral spine-deformity, the medical device 1 is operated by an orthopedic surgeon (hereinafter referred to as "the operator") in advance of surgery. With the medical device 1, a part of a patient's body is incised for installing a screw, a rod is connected to the screw, and an operational tool such as a hook is manipulated to perform the surgery.

As shown in FIGS. 1 and 2, the medical device 1 includes a base 10 having a holding portion 5 that holds a patient 2 in a prone position. This base 10 includes a substantially rectangular frame 11.

The frame 11 has a pair of right and left longitudinal rails 13, 13 extending in a front-rear direction and arranged in parallel, and a pair of lateral rails 15, 15 extending in a right-left direction and being arranged in parallel, the lateral rails 15, 15 being placed at the front and rear of the longitudinal rails 13, 13 with predetermined space.

Each lateral rail 15 is attached to the longitudinal rail 13 via a connecting member 16 allowing the lateral rail 15 to slide as indicated by arrows in FIG. 1. Accordingly, each lateral rail 15 is shiftable along the longitudinal rail 13.

This connecting member 16 is provided with a fixed-released mechanism for fixing the lateral rail 15 to the longitudinal rail 13 or releasing the lateral rail 15 to be shiftable to the longitudinal rail 13. In this fixed-released mechanism, for example, a lever 18, having an eccentric cam (not shown), is pivotably supported by the connecting member 16, and by displacing the eccentric cam through rotation of the lever 18, the longitudinal rail 13 is held by the connecting member 16. Further, by releasing the eccentric cam away from the longitudinal rail 13, the connecting member 16 is fixed to the longitudinal rail 13, and the connecting member 16 can be released from the longitudinal rail 13. Then, after releasing the connecting member 16 from the longitudinal rail 13 and sliding the lateral rail 15 along the longitudinal rail 13, the connecting member 16 is fixed to the longitudinal rail 13, thereby enabling the lateral rail 15 to be optimally positioned.

In the present embodiment, the lateral rail 15 is fixed to the longitudinal rail 13 by rotational operation of the lever 18. However, the fixed method should not be determined by any specific method. The fixation is performable, for example, with general screws.

A holding portion 5 includes a chest unit 5A for holding the chest of a patient 2, a pelvic unit 5B for holding the pelvis of the patient 2, and a leg unit 5C for holding the legs of the patient 2.

The chest unit 5A is installed to the upper portion of a lateral rail 15a arranged in front. The pelvic unit 5B is installed and positioned between the lateral rails 15, 15 arranged in front and in the rear, the pelvic unit 5B being bridged between the longitudinal rails 13, 13. Further, the leg unit 5C is installed to the upper portion of the lateral rail 15b arranged in the rear.

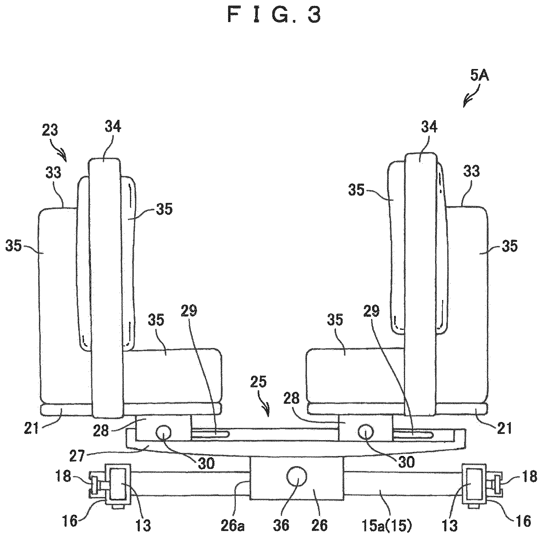

As shown in FIGS. 1 and 3, the chest unit 5A includes a pair of support bodies 21, 21 for respectively supporting the left and right chests of the patient 2, and a holding body 23 being attached to the support body 21 and holding the both shoulders of the patient 2.

The support body 21 is installed to a slider 25 (for chest unit 5A) slidably attached to the lateral rail 15a, the slider 25 being provided with a through hole 26a into which the lateral rail 15a is inserted. This slider 25 includes a pedestal 26 being slidably installed to the lateral rail 15a and a slider main body 27 (for the chest unit 5A) being installed on the pedestal 26.

The lower surface of the slider main body 27 is formed in an arc showing an arrow shape while the upper surface of the pedestal 26 has a curved surface formed according to the arc curvature of the slider main body 27.

The chest unit 5A is made turnable at predetermined curvature by shifting the slider main body 27 on the pedestal 26 laterally (in a right-left direction) in which to allow the chest of the patient 2 to twist. Further, the pedestal 26 is shiftable along the lateral rail 15a enabling the chest portion of the patient 2 to shift in a right-left direction.

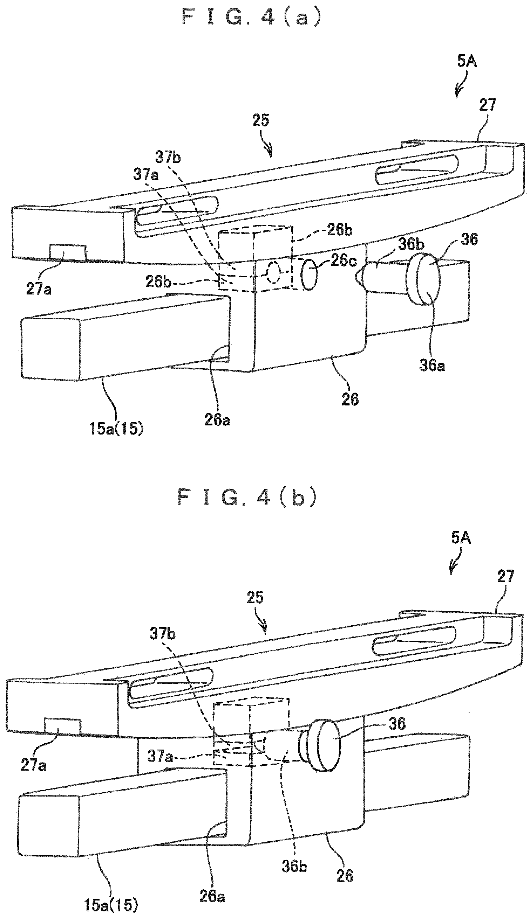

As shown in FIG. 4, the lower surface of the slider main body 27 has a concave portion 27a. Between the concave portion 27a and the pedestal 26, a fixed-released mechanism is provided, the fixed-released mechanism being able to fix movement of the slider main body 27 and the pedestal 26 or being able to release the fixed state thereby enabling the slider main body 27 and the pedestal 26 to shift.

As shown in FIG. 4, this fixed-released mechanism includes, for example, two engaging members 37a, 37b formed from the upper surface of the pedestal 26 in a vertical direction and piled up in a rectangular hole 26b communicating with a through hole 26a in a vertical direction. The fixed-released mechanism also includes a screw 36 (for chest unit 5A) for separating these two engaging members 37a, 37b in the vertical direction.

Among the engaging members 37a, 37b, the lower surface of the engaging member 37a is arranged so as to be in contact with the upper surface of the lateral rail 15a while the upper surface of the engaging member 37b is in contact with the concave portion 27a of the slider main body 27.

Further, as shown in FIG. 4 (a), the screw 36 has a head portion 36a working as a knob and a shaft portion 36b, this shaft portion 36b being inserted into a hole 26c formed on the side surface of the pedestal 26. The shaft portion 36b has a tapered shape, and the tip portion thereof abuts against the boundary of the two engaging members 37a, 37b enabling those two engaging members 37a, 37b to separate to each other.

According to the fixed-released mechanism thus configured, as shown in FIG. 4 (a), when the screw 36 is not inserted into the hole 26c, due to the load of the patient 2, the slider main body 27 comes into contact with the upper surface of the other engaging member 37b while the upper surface of the lateral rail 15a comes into contact with the lower surface of the one engaging member 37a. Further, by making those two engaging members 37a, 37b come into contact to each other, a shift of the slider main body 27 and the pedestal 26 will be restricted (fixed).

On the other hand, as shown in FIG. 4 (b), when the screw 36 is inserted into the hole 26c and the tip thereof abuts against the boundary of the two engaging members 37a, 37b, those two engaging members 37a, 37b are vertically separated. Thus, contact resistance between each of the engaging members 37a, 37b decreases, so that it is possible to shift the slider main body 27 in a right-left direction or to shift the pedestal 26 along the lateral rail 15a in a right-left direction.

In the present embodiment, the pedestal 26 and the slider main body 27 are fixed or released with the fixed-released mechanism, but the pedestal 26 and the slider main body 27 may be fixed or released by using other fixed-released mechanisms.

Further, as shown in FIG. 3, the left and right side of the slider main body 27 has an elongated groove 29 extending in the right-left direction. Each of the support bodies 21 has a projected portion 28 projecting downward, and this projected portion 28 is engageably installed into the elongated groove 29 enabling each support body 21 to freely shift within the elongated groove 29.

Further, the projected portion 28 is installed in such a manner as to hold the upper surface of the slider main body 27, and the side surface of the projected portion 28 is provided with a screw 30 (for chest unit 5A) for adjusting holding force to the slider main body 27. Accordingly, the projected portion 28 holds and is fixed to the slider main body 27 with tightening by which the screw 30 is rotated. Further, the fixed state of the projected portion 28 to the slider main body 27 can be released by loosening the tightening of the screw 30, so that the projected portion 28 can freely shift. Position of each support body 21 can be thus adjusted by shifting the projected portion 28 within the elongated groove 29.

In this manner, each of the support bodies 21, 21 is installed to the slider main body 27 in such a manner as to be slidable in a right-left direction, and width between the support bodies 21, 21 can be adjusted by shifting each of the support bodies 21 and 21.

As shown in FIG. 1, the holding body 23 is provided with: a standing body 33 standing at the end portion of the support body 21 and holding the armpit of the patient 2; and an annular body 34 being integrally provided with the support body 21 and holding the outer shoulder of the patient 2, the annular body 34 being placed inside of the standing body 33. The arm of the patient 2 is then inserted between the standing body 33 and the annular body 34, so that the area around the shoulder of the patient 2 can be held.

Each elastic pad 35 is provided on the upper part of the support body 21, the periphery of the standing body 33, and the front inner side of the annular body 34, so that the area around the shoulder as well as the chest parts of the patient are fixed. Preferably, the pad 35 is made of a radiolucent material. Further, by clasping the body of the patient 2 with the pad 35, even if there are some measurement errors in width between the support bodies 21, 21, the chest of the patient 2 can be securely held.

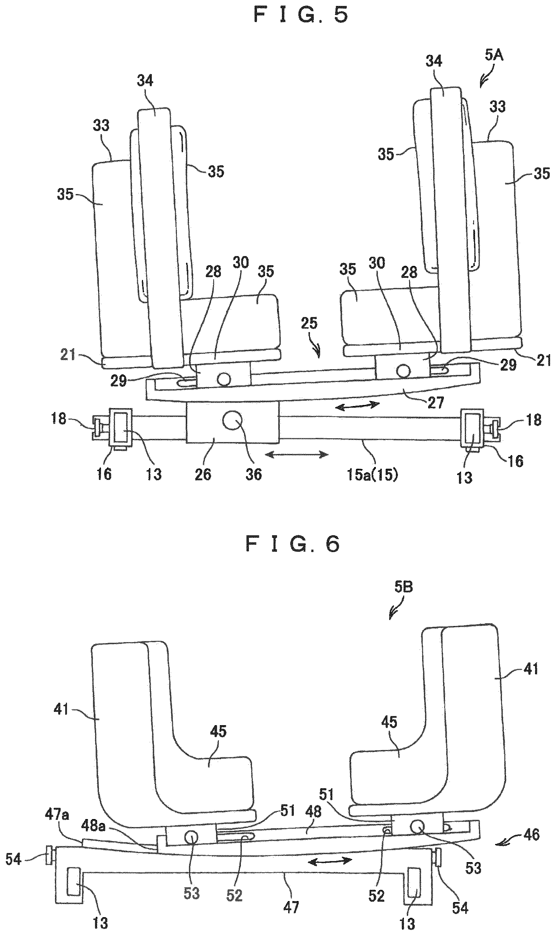

In this manner, as shown in FIG. 5, the chest unit 5A can hold the chest of the patient and can shift it in a right-left direction or make it turned (twisted) as indicated by arrows in FIG. 5. Thus, depending on the state of spinal deformity, the operator will twist and laterally (in a right-left direction) shift the patient's body.

Next, as shown in FIGS. 1, 2 and 6, the pelvic unit 5B has a pair of supports 41, 41 formed in a substantially L shape for respectively supporting the left and right pelves of the patient 2, the supports 41, 41 being arranged so as to face each other. With the supports 41, 41, the area around the pelvis of the patient are held.

As shown in FIG. 6, the supports 41, 41 are installed into a slider 46 (for pelvic unit 5B) bridged between the longitudinal rails 13, 13. This slider 46 comprises: a stand 47 fixedly attached to the left and right longitudinal rails 13, 13; and a slider main body 48 (for pelvic unit 5B) attached to the stand 47.

The lower surface of the slider main body 48 is formed into an arc showing an arrow shape while the upper surface of the stand 47 has a curved surface formed according to the arc curvature of the slider main body 48. Further, the lower surface of the slider main body 48 has a concave portion 48a while the upper surface of the stand 47 has a convex portion 47a that engages with the concave portion 48a. The slider main body 48 can thus shift (turn) on the stand 47 in a right-left direction.

In addition, the slider 46 is provided with a turning mechanism (not shown) for turning the slider main body 48 in a right-left direction with respect to the stand 47. Although not shown, the turning mechanism includes: for example, a first screw that is provided so as to be exposed at a part of the convex portion 47a formed on the stand 47; and a second screw that is screwed with the first screw and is provided in the concave portion 48a of the slider main body 48. The rotation of the second screw will enable the slide main body 48 to shift.

Further, the right and left side surfaces of the stand 47 each has a knob 54 that rotates the first screw. By rotating the knob 54, the first screw will rotate, and this causes the second screw also to rotate. With this structure, the slide main body 48 will slidably shift in a right-left direction and turn on the stand 47, as indicated with an arrow in FIG. 6.

Still further, by shifting the slider main body 48 on the stand 47 in a right-left direction, the pelvic unit 5B is allowed to turn at predetermined curvature. This makes it possible to twist the patient's pelvis.

In the present embodiment, since emphasis is placed on pelvic alignment, the pelvic unit 5B will first position the pelvis of patient. Then, after fixing the pelvis, spine correction for a patient will be performed by using the chest unit 5A or the leg unit 5C.

As shown in FIG. 6, the right and left of the slider main body 48 each has an elongated groove 52 (for pelvic unit 5B) extending in the right-left direction, and each of the supports 41, 41 has a projected portion 51 (for pelvic unit 5B) projecting downward, the projected portion 51 engaging with and being installed to the elongated groove 52. This allows each of the supports 41, 41 to freely shift within the elongated groove 52.

The projected portion 51 is installed in such as manner as to hold the upper surface of the slider main body 48. The side surface of each projected portion 51 is provided with a screw 53 that adjusts holding force of the projected portion 51 to the slider main body 48. Through tightening by the rotation of the screw 53, the projected portion 51 will hold and be fixed to the slider main body 48. On the other hand, by releasing the holding force through the rotation of the screw 53, the fixed state of the projected portion 51 to the slider body 48 can be released, allowing the projected portion 51 to freely shift (See FIG. 6).

Then, by shifting the projected portion 51 within the range of the elongated groove 52, the position of the supports 41, 41 can be adjusted.

In this manner, the respective support 41, 41 is installed to the slider main body 48 enabling each support 41, 41 to shift in a right-left direction. Accordingly, depending on the body shape of a patient, each support 41, 41 is shifted so as to adjust width between the supports 41, 41.

The support 41 has an elastic pad 45 for holding the pelvis of the patient 2. This pad 45 is preferably made of a radiolucent material. Further, by clasping the body of the patient 2 with the pad 45, even if there are some measurement errors in width between the supports 41, 41, the pelvis of the patient 2 can be securely held.

In this manner, the pelvic unit 5B will hold the pelvis of the patient 2 and turn (twist) the pelvis in a right-left direction. Depending on the state of spinal deformity, the operator twists the pelvis of the patient 2 so as to position and fix the pelvis of the patient.

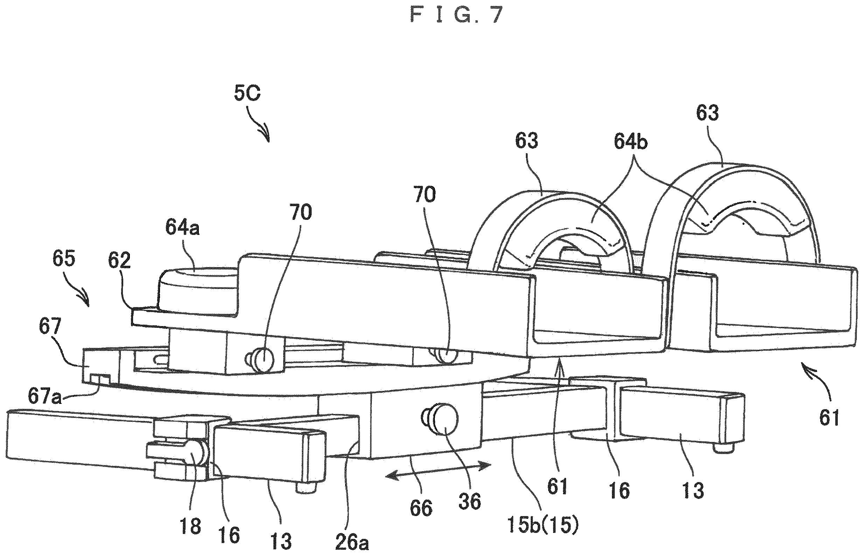

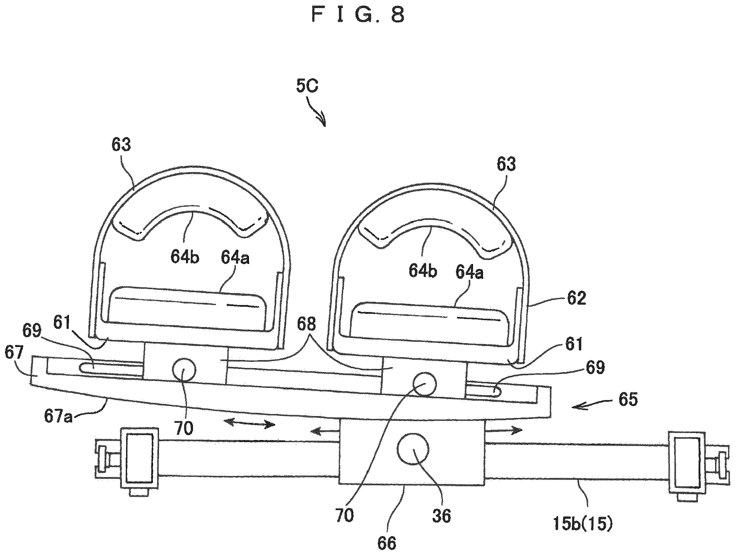

Next, as shown in FIGS. 7 and 8, the leg unit 5C includes a pair of supporting units 61, 61 which support the left and right lower leg portions of the patient 2, and the lower leg portions are thus held by each supporting unit 61. The supporting unit 61 is installed to a slider 65 (for leg unit 5C) slidably attached to the lateral rail 15b. This slider 65 includes a seat 66 slidably attached to the lateral rail 15b and a slider main body 67 (for leg unit 5C) mounted on the seat 66.

The lower surface of the slider main body 67 is formed in an arc showing an arrow shape while the upper surface of the seat 66 has a curved surface formed according to the arc curvature of the slide main body 67.

The leg unit 5C is turnable at predetermined curvature by shifting the slider main body 67 on the seat 66 laterally (in a right-left direction) allowing the legs of the patient 2 to twist. Further, the seat 66 is shiftable along the lateral rail 15b enabling the legs of the patient 2 to shift in a right-left direction (see FIG. 8).

Further, the lower surface of the slider main body 67 has a concave portion 67a. Between the concave portion 67a and the seat 66, a fixed-released mechanism is provided, the fixed-released mechanism being able to fix movement of the slider main body 67 and the seat 66 or being able to release the fixed movement making the slider main body 67 and the seat 66 shiftable.

Since this fixed-released mechanism is adopting the same mechanism as that of the aforementioned chest unit 5A, its explanation is omitted. In this embodiment, the seat 66 and the slider main body 67 are fixed or released by the fixed-released mechanism, but the seat 66 and the slider main body 67 may be fixed or released by using other fixed-released mechanisms.

Further, as shown in FIG. 8, the left and right side of the slider main body 67 each has an elongated groove 69 (for leg unit 5C) extending in the right-left direction. Each of the supporting units 61 has a projected portion 68 (for leg unit 5C) projecting downward, and this projected portion 68 is engageably installed into the elongated groove 69 allowing each supporting unit 61 to freely shift in the elongated groove 69.

Further, the projected portion 68 is installed in such a manner as to hold the upper surface of the slider main body 67, and the side surface of the projected portion 68 is provided with a screw 70 for adjusting the holding force. Accordingly, the projected portion 68 holds and is fixed to the slider main body 67 through tightening by which the screw 70 is rotated. Further, the fixed condition of the projected portion 68 to the slider main body 67 can be released by loosening the tightening of the screw 70, so that the projected portion 68 can freely shift.

Position of each supporting unit 61 can be thus adjusted by shifting the projected portion 68 in the elongated groove 69.

Accordingly, each of the supporting units 61, 61 are installed to the slider main body 67 in such a manner as to be slidable in a right-left direction, whereby width between the supporting units 61, 61 is adjusted by shifting each of the supporting units 61, 61.

As shown in FIGS. 1, 7, and 8, each supporting unit 61 includes a plate-shaped base body 62 for holding the anterior of each lower leg of the patient 2, and on the base end side of the base body 62 is provided with an elastic pad 64a (for leg unit 5C) on which each knee portion of the patient 2 is placed. And, the top end side of the base body 62 has an annular member 63 integrally provided with the base body 62 and holding the ankle portion of the patient 2. Further, on the inner side of each annular member 63 has an elastic pad 64b. Preferably, those pads 64a, 64b are made of a radiolucent material. In addition, by clasping the body of the patient 2 with the pads 64a, 64b, even if there are some measurement errors in width between the supporting units 61, 61, each leg (lower leg portion) of the patient 2 can be securely held.

In this manner, the leg unit 5C can hold the legs of the patient, can shift them in a right-left direction, or turn (twist) them. Thus, depending on the state of spinal deformity, the operator will twist the legs of the patient 2 and also shift them in a right-left direction.

In addition, as shown in FIG. 1, the medical device 1 is detachably provided with a compression correction device 80, the compression correction device 80 compressing a part of a vertebra constituting a spinal column placed between the chest unit 5A and the pelvic unit 5B.

The compression correction device 80 includes a standing body 81 attached to the longitudinal rail 13 and standing in a vertical direction, and an extrusion body 85 attached to the standing body 81 via a connector 83 and extrudable in the right-left direction.

The standing body 81 is detachably installed to the longitudinal rail 13 via a connecting part 82. An elastic pad 87 (for compression correction device 80) is provided at the top end portion of the extrusion body 85, this pad 87 compressing a part of the vertebra constituting the spinal column.

Then, the extrusion body 85 is provided slidably in a vertical direction via the connector 83, the extrusion body 85 being thus adjustable in the vertical direction. Further, the pad 87 is provided at the tip of the rod-like extrusion body 85 extending in the right-left direction, and the extrusion body 85 is provided shiftably in a right-left direction via the connector 83. This will adjust the horizontal position of the pad 87.

Note that the compression correction device 80 may be provided at a plurality of places, depending on the state of spinal deformity.

As described above, treatment in the present embodiment will be performed while the pelvis of the patient is fixed, and emphasis is placed on pelvic alignment.

Again, in the medical device 1 of the embodiment of the present invention, the pelvic unit 5B is fixedly installed to the longitudinal rail 13, and after positioning the pelvis of the patient, the chest unit 5A is shifted forward so as to pull the spine of a patient. Then by shifting the chest unit 5A in a right-left direction, depending on the state of spinal deformity, the spine will be corrected.

Next, the specific usage of the medical device 1 will be exemplified.

First, the patient 2 is placed in a prone position on the chest unit 5A, the pelvic unit 5B, and the leg unit 5C, which are arranged side by side on the base 10. In this condition, depending on the body type of the patient 2, by using various screws 30, 53, and 70, the space between the support bodies 21, 21 of the chest unit 5A, the space between the supports 41, 41 of the pelvic unit 5B, and the space between the supporting units 61, 61 of the leg unit 5C are adjusted so as to make each support body 21, support 41, and supporting unit 61 fit to the patient 2.

Next, the pelvic unit 5B will fix (position) the pelvis of the patient 2. Then, depending on the body type of the patient 2, each position of the chest unit 5A and the leg unit 5C is adjusted so as to fix the body of the patient 2.

Next, the chest unit 5A is shifted to separate from the pelvic unit 5B. The chest unit 5A is then held. In this manner, the spine of the patient 2 is pulled.

Then, the chest unit 5A is turned left and right depending on the deformed state of the spine. This allows only the chest of the patient 2 to be compressed in the right-left direction while the pelvic portion of the patient 2 is fixed. This enables to facilitate easier correction for a spine.

Depending on the deformed state of the spine, the compression correction device 80 is installed between the chest unit 5A and the pelvic unit 5B so as to press a part of a vertebra constituting a spine. The spine is then corrected therewith.

### The Second Embodiment

Next, the second embodiment of the medical device will be explained.

In the medical device of the first embodiment, the pelvic unit 5B is fixed, and the chest unit 5A and the leg unit 5C are made shiftable closer to or away from the pelvic unit 5B. The first embodiment only allows the slider 46 of the pelvic unit 5B to turn. However, in the second embodiment, as the same with the chest unit 5A and the leg unit 5C, the pelvic unit 5B is made shiftable along the longitudinal rail 13. Accordingly, the second embodiment is different from the first embodiment in that the slider 46 is not only able to turn but also able to shift in a right-left direction.

Since the structure of the slider 46 of the pelvic unit 5B of the present embodiment is the same as that of the chest unit 5A or the leg unit 5C, its explanation is omitted.

By structuring the pelvic unit 5B in this manner, this makes it possible for the chest unit 5A and the pelvic unit 5B to relatively shift in a right-left direction, or makes it possible for the pelvis of a patient to shift in a right-left direction or to turn while the chest has been fixed. Also, within the range of the longitudinal rail 13, the patient can freely shift while being fixed.

### The Third Embodiment

Next, the third embodiment of a medical device will be described.

In the medical device of the present embodiment, although the medical device of the first or the second embodiment allows the chest unit 5A, the pelvic unit 5B or the leg unit 5C to turn, this third embodiment only allows the chest unit 5A, the pelvic unit 5B or the leg unit 5C to shift in a right-left direction. The third embodiment is different from the first and the second embodiments in this regard.

The chest unit 5A and the pelvic unit 5B of the present embodiment can be achieved by integrally fixing the slider main bodies 27, 48 and the pedestal 26 and the stand 47, as shown in FIGS. 3 or 6. Since the other structures are the same, its explanation is omitted.

By structuring the chest unit 5A, the pelvic unit 5B or the leg unit 5C in this manner, although incapability of turning will restrict a corrective range for a patient, its manufacturing cost can be greatly reduced.

### The fourth embodiment

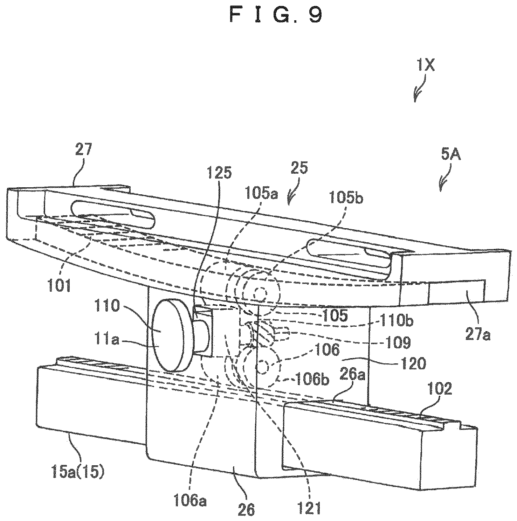

Next, the fourth embodiment of the medical device 1 will be explained with reference to FIG. 9.

A medical device 1X of the present embodiment is another example of the fixed-released mechanism provided in the chest unit 5A and the leg unit 5C of the medical device 1 of the first embodiment. Although the fixed-released mechanism below is explained for the one using for the chest unit 5A, it is applicable also for the leg unit 5C.

As shown in FIG. 9, the fixed-released mechanism of the present embodiment includes two gears 105, 106 which are formed in the vertical direction from the upper surface of the pedestal 26 and arranged in a space 120 communicating with a through hole 26a, and a screw 110 connected to the two gears 105, 106. Further, the gears 105, 106 are provided with worm gears 105a, 106a and helical gears 105b, 106b arranged coaxially.

One worm gear 105a engages with a rack gear 101 formed on the lower surface of the slider main body 27 while the other worm gear 106a engages with a rack gear 102 formed on the upper surface of the pedestal 26.

The screw 110 has a head portion 110a functioning as a knob and a shaft portion 110b. This shaft portion 110b is inserted into a long hole 121 extending in the vertical direction, the long hole 121 being formed on the side surface of the pedestal 26 communicating with the space 120. Further, the tip of the shaft portion 110b is provided with a helical gear 109 that engages with the helical gears 105b, 106b of each gear 105, 106.

In addition, the screw 110 is disposed in the long hole 121 via a positioning element 125 arranged on the outer peripheral surface of the shaft portion 110b. By shifting the positioning element 125 in the long hole 121 in a vertical direction, the screw 110 can be shifted to any position where desired.

As the screw 110 shifts upward, it engages with the worm gear 105a on the slider main body 27 side while it engages with the worm gear 106a on the pedestal 26 side as the screw 110 shifts downward.

Therefore, the screw 110 shifts either in the upper direction or in the lower direction thereby transmitting its rotational driving force to one of the worm gear 105a or 106a through the rotation of the head portion 110a. With this structure, the driving force will transfer via one of the rack gears 101, 102 so as to enable the slider main body 27 or the pedestal 26 to shift.

Note that the screw 110 may shift toward a central position where the helical gear 109 of the screw 110 does not engage with the helical gears 105b, 106b of the gear 105, 106. With the screw 110 shifting to the central position, it can prevent the slide main body 27 and the pedestal 26 to shift in error.

In this embodiment, since the slider main body 27 or the pedestal 26 can shift via gears and the like, it is possible to easily position the patient 2 even after the patient 2 has been already placed on the medical device 1.

As described, in the medical device 1 according to the present embodiment, the chest unit 5A that supports the chest of the patient 2, the pelvic unit 5B that supports the pelvis of the patient 2, and the leg unit 5C that supports the legs of the patient 2 are arranged side by side on the base 10. Then, while the patient 2 is held in a prone position with the chest unit 5A, the pelvic unit 5B and the leg unit 5C, the spine/ joint of the patient 2 will be treated. To accomplish the above, the medical device 1 includes: the lateral rail 15 that enables the chest unit 5A or the leg unit 5C to be displaced relative to the pelvic unit 5B in a horizontal direction and that is slidably provided to the longitudinal rail 13 via the connecting member 16 shiftable in a first direction; and the sliders 46, 25 that enable the pelvic unit 5B or the chest unit 5A to shift in a horizontal direction orthogonal to the first direction.

Further, the chest unit 5A or the leg unit 5C is provided with the lever 18 as the fixed-released mechanism for fixing or releasing the lateral rail 15 relative to the longitudinal rail 13.

In such a medical device 1, after the chest unit 5A or the leg unit 5C has shifted so as to separate from the pelvic unit 5B and has been held, and after the spine of the patient has been pulled, the chest unit 5A or the pelvic unit 5B shifts in a right-left direction or turns according to the deformed state of the spine. This makes it possible to easily correct the deformity of the spine.

Since the medical device 1 is structured with a simple mechanism as described above, it is possible to reduce the manufacturing cost without enlarging the size thereof and to obtain good corrective operability of spinal deformity.

### The Fifth Embodiment

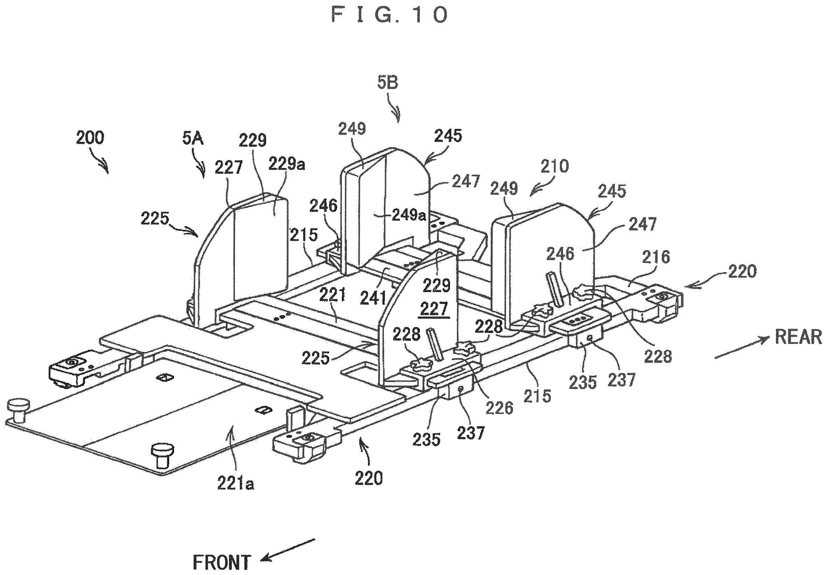

Next, the fifth embodiment of the medical device will be explained with reference to FIGS. 10 to 12.

A medical device 200 in the present embodiment shows an example that the leg unit 5C of the medical device 1 in the first embodiment is eliminated, and the chest unit 5A and the pelvic unit 5B are simplified.

Specifically, the medical device 200 is the same with the first embodiment in that the chest unit 5A and the pelvic unit 5B are shiftable in a front-back direction; however, they are different in that the pair of support bodies 21, 21 of the chest unit 5A and the pair of supports 41, 41 of the pelvic unit 5B of the present embodiment are shiftable as one body in a right-left direction while a pair of support bodies 227, 227 of the chest unit 5A and a pair of supports 247, 247 of the pelvic unit 5B are individually shiftable in a right-left direction. Further, the fifth embodiment is also different from the first embodiment in that it does not have a rotational mechanism.

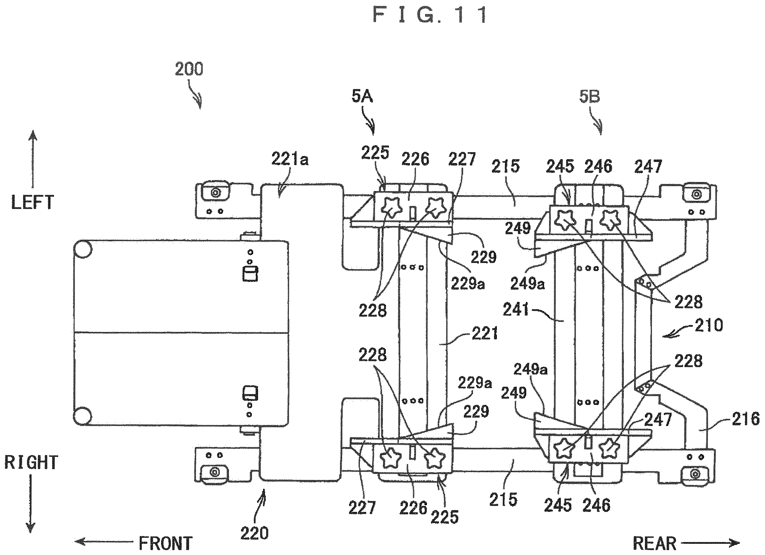

As shown in FIGS. 10 and 11, the medical device 200 of the present embodiment includes, as an example, a base 220 having a holding area 210 for holding the patient 2 in a prone position. The medical device 200 is used in that it is set on a bed device such as a surgical table (not shown) generally used. Although the medical device 200 is fixed to the bed device, any fixing device may be applied as long as the medical device 200 can be fixed to the bed device. Since the fixing mechanism thereof can be appropriately selected from well-known techniques, the description thereof will be omitted.

The base 220 includes a pair of left and right longitudinal rails 215, 215 extending in a front-rear direction and arranged in parallel to each other, and a lateral rail 216 which is bridged between the longitudinal rails 215, 215.

The holding area 210 includes a chest unit 5A that holds the chest of the patient 2 and a pelvic unit 5B that holds the pelvis of the patient 2. The chest unit 5A is arranged in the anterior portion of the pair of longitudinal rails 215, 215 while the pelvic unit 5B is arranged substantially on the same plane behind the chest unit 5A.

The chest unit 5A includes a base substrate 221 on which the chest of the patient 2 is placed, and a holder 225 installed to the left and right of the base substrate 221 and holding the side of the chest of the patient 2.

The base substrate 221 is slidably installed to each of the longitudinal rails 215, 215 via a connecting body 235 in such a manner as that a flat plate member is bridged between the longitudinal rails 215, 215. This base substrate 221 has an extension 221a extending forward, the extension 221a allowing the shoulder or the face of the patient 2 to be placed.

The connecting body 235 is arranged so as to surround the circumferential edge of the longitudinal rail 215, and the side surface of the connecting body 235 is provided with a fixture 237 for fixing the connecting body 235 to the longitudinal rail 215.

Although not shown, the fixture 237 includes a rotatable head portion and a shaft portion extending vertically downward from the head portion. The fixture 237 is installed by being screwed to a hole (not shown) formed on the side surface of the connecting body 235. By fastening the fixture 237 and bringing the tip of the shaft portion into contact with the side surface of the longitudinal rail 215, the connecting body 235 is fixed to the longitudinal rail 215. Note that, by loosening the head of the fixture 237 by rotating it in a reverse direction and shifting the tip of the shaft portion away from the side surface of the longitudinal rail 215, the connecting body 235 can freely shift with respect to the longitudinal rail 215. Further, the fixture 237 may be installed to the left and right connecting bodies 235, respectively, or may be installed to only one of them.

Further, the holder 225 includes a pair of base stands 226, 226 attached to the left and right sides of the base substrate 221 so as to be slidable in a width direction (in a right-left direction), and support bodies 227, 227 standing on each of the base stands 226, 226 and supporting the lateral sides of the chest of the patient 2 (shoulder-joints of both arms or armpit). With this structure, certain pressure is applied to the patient 2 with the left and right support bodies 227, 227 so as to support the both sides of the chest of the patient 2.

For the support body 227, a plate-shaped member having a size sufficiently supporting the lateral side of the chest of the patient 2 is used. However, when performing corrective treatment for a spine with the medical device 200 of the present embodiment, the support body 227 will also function as a pressing body that compresses one side portion of the patient 2. Accordingly, the support body 227 will be not sufficient enough only if it enables to support the chest of the patient 2, but the support body 227 at least needs to have height corresponding to the thickness of the chest of the patient 2, and necessary strength. The height and strength of the support body 227 are thus suitably set.

At the back of the support body 227 is formed with a projection 229 that projects inward. This projection 229 is provided with an inclined surface 229a that gradually reduces width between the support bodies 227, 227 from the center to the rear of the support bodies 227, 227 in the longitudinal direction. The distance between the support bodies 227, 227 is thus made in that the width of the rear is narrower than the width of the front.

In addition, the base stand 226 is provided with an adjustment mechanism for adjusting the installation position of each support bodies 227 with respect to the base substrate 221 according to the body shape of the patient 2, and this adjustment mechanism is installed to the front and rear upper surfaces of the base stand 226.

This adjustment mechanism is, for example, a fixing tool 228 having a rotatable head portion and a shaft portion extending vertically downward from the head portion. The adjustment mechanism is installed in such a manner as to screw to a hole portion (not shown) formed on the upper surface of the base stand 226. Then, by making the tip of the shaft portion abutting to the upper surface of the base substrate 221 while fastening the fixing tool 228, the base stand 226 can be fixed to the base substrate 221. On the other hand, by inversely rotating and loosing the fixing tool 228, it is possible for the tip of the shaft portion to release from the upper surface of the base substrate 221, allowing the base stand 226 to freely shift relative to base substrate 221.

The pelvic unit 5B includes a substrate 241 on which the pelvis of the patient 2 is placed, and a holding unit 245 (for pelvic unit 5B) attached to the left and right of the substrate 241 and holding the side of the pelvis of the patient 2.

The substrate 241 is slidably installed to each of the longitudinal rails 215, 215 via the connecting body 235 so that a flat plate member is bridged between the longitudinal rails 215, 215.

Since a connecting body 235 and a fixture 237 installed to the connecting body 235 are the same structure as the connecting body 235 provided in the chest unit 5A, the explanation thereof will be omitted.

Further, a holding unit 245 includes a pair of base portions 246, 246 attached to the left and right sides of the substrate 241 so as to be slidable in a width (right-left) direction, and supports 247, 247 (for pelvic unit 5B) standing on each of the base portions 246 and supporting the lateral side of the pelvis of the patient 2. With this structure, certain pressure is applied to the patient 2 with the left and right supports 247, 247 so as to support the both sides of the pelvis of the patient 2.

For the support 247, a plate-shaped member having a size sufficiently supporting the lateral side of the pelvis of the patient 2 is used. However, when performing a spine correction treatment by using the medical device 200 of the present embodiment, the support 247 will also function as a pressing body that compresses one side portion of the patient 2. Accordingly, the support 247 will be not sufficient enough only if it enables to support the pelvis of the patient 2, but the support 247 at least needs to have the height corresponding to the thickness of the pelvis of the patient 2 and necessary strength. The height and the strength of the support 247 are thus suitably set.

At the front of the support 247 is formed with a projection 249 that projects inward. This projection 249 is provided with an inclined surface 249a that gradually reduces width between the supports 247, 247 from the center to the front of the supports 247, 247 in its longitudinal direction. The distance between the supports 247, 247 is thus made in that the width of the front is narrower than the width of the rear.

In addition, the base portion 246 is provided with an adjustment mechanism for adjusting the installation position of each support 247 with respect to the substrate 241 according to the body shape of the patient 2, and this adjustment mechanism is installed to the front and rear upper surfaces of the base portion 246.

Since the adjustment mechanism is the same structure with the fixing tool 228 provided to the chest unit 5A discussed hereinabove, the detail explanation is omitted.

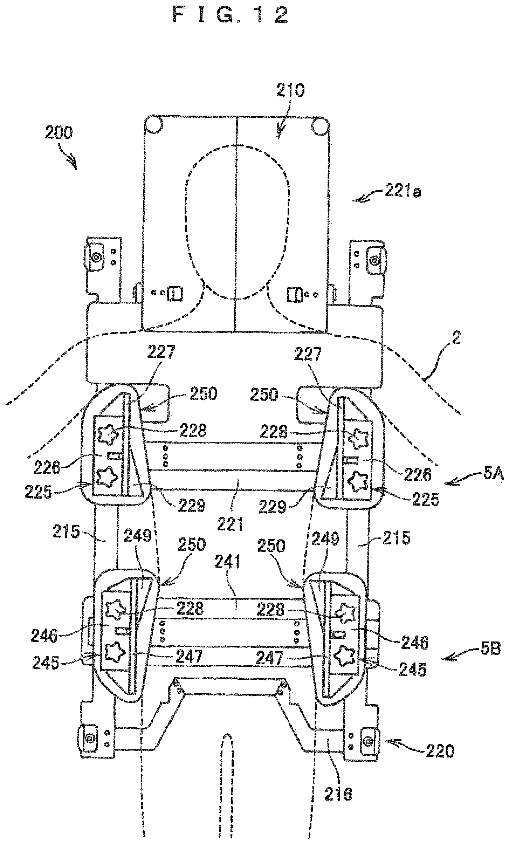

Further, as shown in FIG. 12, in order to alleviate contact with the body of the patient 2, the support body 227 and support 247 of the chest unit 5A and the pelvic unit 5B are provided with a cushioning material 250 having predetermined thickness, the cushioning material 250 being provided so as to surround each support body 227 and support 247. The cushioning material 250 is disposed at least in the area of the support body 227 and support 247 that will contact the body of the patient 2.

Note that the cushioning material 250 may also be disposed on the top surface of the base substrate 221 and the substrate 241. Further, when the support body 227 and the support 247 are each made of an elastic body, it is not necessary to provide the cushioning material 250. In this case, the cushioning material 250 is provided if necessary.

As explained above, the medical device 200 of the present embodiment is a medical device in which the chest unit 5A for supporting the chest of the patient 2 and the pelvic unit 5B for supporting the pelvis of the patient 2 are arranged side by side on the base 220. By fixing the pelvic unit 5B on the base 220 and loosening the fixture 237 installed to the connecting body 235 provided in the chest unit 5A, the chest unit 5A can freely shift back and forth. This will allow the chest unit 5A to shift closer to or separating from the pelvic unit 5B, which makes performance of the pulling operation to the patient easier. Further, in the medical device 200, by fixing the chest unit 5A and the pelvic unit 5B on the base 220 and loosening the fixing tool 228, the base stand 226 and the base portion 246 can freely shift in a width direction of the base substrate 221 and the substrate 241. Accordingly, by fixing one of the chest unit 5A and the pelvic unit 5B and by shifting the other in a width direction, treatment to correct spinal deformity can be easily performed.

In addition, since the projections 229, 249 are provided on the support body 227 and the support 247 of the chest unit 5A and the pelvic unit 5B, when performing the pulling operation to the patient, it is possible to easily increase contact pressure between the body of the patient 2 and the support body 227/support 247. This will prevent the patient 2 from being slipped with respect to the support body 227 and the support 247 thereby enabling to surely perform the pulling operation. Further, in this embodiment, the projections 229, 249 are determined to be the inclined faces 229a, 249a, but the shape thereof is not limited thereto. Any form may be adopted as long as the slippage prevention effect is obtainable.

Moreover, in the present embodiment, the support body 227 and the support 247 are provided with the projections 229, 249. Accordingly, when performing the pulling operation to a patient, it can increase contact pressure between the body of the patient 2 and the support body 227/support 247. This however may also be achieved by: forming the support body 227 and the support 247 with curvature; or by installing the support body 227 and the support 247 to the base stand 226 and the base portion 246 to be rotatable around a shaft in a vertical direction and then fixing the support body 227 and the support 247 with a predetermined angle.

Next, specific examples of how to use the medical device 200 will be described.

First, the patient 2 is placed on the chest unit 5A and the pelvic unit 5B in a prone state, the chest unit 5A and the pelvic unit 5B being arranged side by side on the base 220. In this condition, depending on the body type of the patient 2, the adjusting mechanism will adjust the space between the holders 225 of the chest unit 5A and the space between the holding units 245 of the pelvic unit 5B. Accordingly, the respective support body 227 and the support 247 are adjusted so as to enable them to fit to the patient 2 for holding the body of the patient. When fixing the chest unit 5A and the pelvic unit 5B, the pelvis of the patient 2 is first fixed (positioned) by using the pelvic unit 5B, and then the position of the chest unit 5A is adjusted to fix a patient.

Next, the chest unit 5A is shifted for separating it from the pelvic unit 5B, and the chest unit 5A is then held. Through this, the spine of the patient 2 can be pulled.

Then, depending on the deformed state of a spine, one of the pelvic unit 5B or the chest unit 5A is uni-directionally pressed to either left or right of the other so as to perform the spine correction. At this time, either one of the support body 227 or the support 247 is fixed while the fixation of the other is released. In this condition, the support body 227 or the support 247 of the other is pressed in one direction to perform the corrective treatment.

As described above, although simply structured, the medical device 200 of this embodiment can easily perform corrective treatment of a spine, including those pulling performances. In addition, since it has a simple structure, it is possible to easily increase productivity and to manufacture the medical device 200 at low cost.

Although the pair of support bodies 227, 227 or the pair of supports 247, 247 of the present embodiment independently shift in a width direction, as shown in the first embodiment, the pair of support bodies 227, 227 or the pair of supports 247, 247 may link to each other and uni-directionally shift either on the right or on the left.

Note that this embodiment is only one mode and not limited thereto. For example, each adjustment mechanism of the present embodiment may adopt a known technique regardless of the one not described hereinabove. In addition, Embodiments 1 to 5 may be combined as appropriate.

Further, the structure of the shifting mechanism for shifting the chest unit 5A or the pelvic unit 5B in a front-rear direction with respect to the base 220 is not limited to these embodiments, but any known technique may be adopted. Also, the shifting mechanism for shifting the holder 225 and the holding unit 245 in a width direction as well as the mechanism for fixing or releasing the holder 225 and the holding unit 245 to the base substrate 221 and the substrate 241 are not limited to these embodiments but may be adopted with any known technique.

### Explanation of References

1 medical device;
2 patient;
5A chest unit;
5B pelvic unit;
5C leg unit;
10 base;
15 lateral rail;
16 connecting member; and
25, 46 slider

## Claims

1. A medical device in which a chest unit supporting the chest of a patient and a pelvic unit supporting the pelvis of the patient are arranged side by side on a base,
wherein the medical device comprises: a first shifting device enabling the chest unit to shift closer to or away from the pelvic unit, the shift being defined as a first direction; and a second shifting device enabling either the chest unit or the pelvic unit to shift to the other in a horizontal direction orthogonal to the first direction.

2. The medical device according to claim 1, further comprising: a first holding device that holds the shifted state made by the first shifting device,
wherein the second shifting device shifts the pelvic unit or the chest unit in a condition that the pelvic unit or the chest unit has shifted and been held by the first shifting device.

3. The medical device according to claim 2, further comprising a second holding device that holds the shifted state made by the second shifting device.

4. The medical device according to any one of claims 1 to 3, wherein the chest unit has a width of a rear portion that is narrower than a width of a front portion thereof in a longitudinal direction, and the pelvic unit has a width of a front portion that is narrower than a width of a rear portion thereof in a longitudinal direction.
